# EUROPEAN PATENT SPECIFICATION

(11) **EP 1 712 284 B1**
(45) Date of publication and mention of the grant of the patent: **10.10.2012**
(21) Application number: 06007857.3
(22) Date of filing: 13.04.2006
(51) Int. Cl.: B01L 3/00

(54) **Cell separation method using hydrophobic solid supports**
Methode zur Zelltrennung mit Benutzung hydrophober fester Träger
Méthode de séparation de cellules comprenant l'utilisation de supports solides hydrophobes

(30) Priority: 15.04.2005 KR 20050031406; 05.09.2005 KR 20050082442
(43) Date of publication of application: 18.10.2006
(73) Proprietor: Samsung Electronics Co., Ltd., Suwon-si, Gyeonggi-do, 443-742 (KR)
(72) Inventor: Lim, Hee-kyun 147-602 Hwanggol Maeul Jugong, Yeongtong-gu Suwon-si Gyeonggi-do (KR); Hwang, Kyu-youn, Incheon-si (KR); Kim, Joon-ho, Bundang-gu Seongnam-si Gyeonggi-do (KR); Namkoong, Kak, Seoul (KR); Park, Chin-sung 311-1601 Hyundai Morningside 1-cha, Gyeonggi-do (KR)
(74) Representative: Grünecker, Kinkeldey, Stockmair & Schwanhäusser

(56) References cited:
- SIA S.K. ET AL: "Microfluidic devices fabricated in poly(dimethylsiloxane) for biological studies" ELECTROPHORESIS, vol. 24, 2003, pages 3563-3576, XP002384096
- REYES D R ET AL: "Micro Total Analysis Systems. 1. Introduction, Theory and Technology" ANALYTICAL CHEMISTRY, AMERICAN CHEMICAL SOCIETY. COLUMBUS, US, vol. 74, 2002, pages 2623-2636, XP002311567 ISSN: 0003-2700
- AUROUX P-A ET AL: "Micro total analysis systems. 2. Analytical standard operations and applications" ANALYTICAL CHEMISTRY, AMERICAN CHEMICAL SOCIETY. COLUMBUS, US, vol. 74, no. 12, 15 June 2002 (2002-06-15), pages 2637-2652, XP002339167 ISSN: 0003-2700
- VILKNER T ET AL: "MICRO TOTAL ANALYSIS SYSTEMS. RECENT DEVELOPMENTS" ANALYTICAL CHEMISTRY, AMERICAN CHEMICAL SOCIETY. COLUMBUS, US, vol. 76, no. 12, 15 June 2004 (2004-06-15), pages 3373-3385, XP002355410 ISSN: 0003-2700
- COX J D ET AL: "Surface passivation of a microfluidic device to glial cell adhesion: a comparison of hydrophobic and hydrophilic SAM coatings" BIOMATERIALS, ELSEVIER SCIENCE PUBLISHERS BV., BARKING, GB, vol. 23, no. 3, February 2002 (2002-02), pages 929-935, XP004348108 ISSN: 0142-9612
- LIPOWSKI AND SACKMANN: "HANDBOOK OF BIOLOGICAL PHYSICS" 1995, ELSEVIER SCIENCE B.V , XP002384118 * page 755 - page 802 *

## Description

### BACKGROUND OF THE INVENTION

### 1. Field of the Invention

The present invention relates to a cell separation method using hydrophobic solid supports.

### 2. Description of the Related Art

The separation of cells from mixtures containing them and unwanted impurities is a challenging problem in the art. This is particularly the case where the cells are present in a culture broth, a biological sample or similar complex mixture, as the methods employed need to allow the high extent of capturing of the target cells intact. This means that the reagents used in cell concentration and separation steps must capture the cells very efficiently over a broad range of cell densities. Also, the reagents used should not interfere with downstream processes using the cells, the recovery of nucleic acid from the cells and/or the processing of the nucleic acid.

Binding of the cells to the solid support may be achieved in any known or convenient manner. For example, non-specific binding of the cells to a solid support may be achieved by appropriately choosing the properties of a solid support and the surrounding conditions e.g., the chemical or physical nature of the surface of the solid support (e.g., hydrophobicity or charge), the pH or composition of the isolation medium, etc. The nature of the target cells may also play a role and it has been shown, for example, that certain hydrophobic cells may be readily bound non-specifically to hydrophobic surfaces, whereas hydrophilic cells may be readily bound to hydrophilic surfaces. Negatively charged cells such as B-lymphocytes have also been observed to have a high degree of non-specific binding to slightly-positively charged surfaces. Thus solid supports having appropriately charged surfaces for binding of a desired cell type may be used. Appropriate buffers may be used as media for cell separation to achieve the conditions appropriate for cell binding by simply placing the solid support and the sample in contact in an appropriate medium. Conveniently, a buffer of appropriate charge, osmolarity etc. may be added to the sample prior to, simultaneously with, or after contact with the solid support.

U.S. Patent No. 6,617,105 discloses a method of isolating nucleic acid from a sample of cells which includes: binding cells in the sample to a solid support coated with cell-binding moieties; and lysing the isolated cells. In this patented method, a glycidyl-histidine modified magnetic bead is used. However, this patented method does not disclose a hydrophobic solid support having a specific water contact angle, which is used in the present invention.

International Publication No. WO 03/102184 A1 discloses a method of separating cells containing target nucleic acid, comprising: contacting a mixture containing cells with a flocculating agent capable of aggregating the cells, wherein the flocculating agent is a polyamine or a cationic detergent, and a solid phase capable of binding the cells; separating the aggregated cells from the mixture using the solid phase; and purifying the target nucleic acid from the cells. In this patent, a magnetic bead is used as a solid support. However, there is no description of a hydrophobic solid support having a specific water contact angle, which is used in the present invention.

SIA S.K. et al: "Microfluidic devices fabricated in poly(dimethylsiloxane) (PDMS) for biological studies" ELECTROPHORESIS, vol. 24, 2003, pages 3563-3576 is a review about microfluidic devices fabricated in PDMS for biological studies. Biological procedures that have been miniaturized into PDMS-based micro-devices include immunoassays, separation of proteins and DNA, as well as sorting and manipulation of cells.

REYES D R ET AL: "Micro Total Analysis Systems. 1. Introduction, Theory and Technology" ANALYTICAL CHEMISTRY, AMERICAN CHEMICAL SOCIETY. COLUMBUS, US, vol. 74, 2002, pages 2623-2636 is a review regarding micro total analysis systems, including an introduction, the theory of miniaturization, and technologies relating to such micro-fluidic devices, disclosing *inter alia* that microfluidic devices have been used to carry out flow cytometry, as well as using PDMS as one substrate of microfluidic devices.

AUROUX P-A ET AL: "Micro total analysis systems. 2. Analytical standard operations and applications" ANALYTICAL CHEMISTRY, AMERICAN CHEMICAL SOCIETY. COLUMBUS, US, vol. 74, no. 12, 15 June 2002, pages 2637-2652 is the continuation of the review of REYES D R ET AL, wherein the analytical standard operations and applications of microfluidic systems are presented. In the section "Analytical Standard Operations, Separation", chromatography, electrophoresis, isoelectric focusing and other separation methods are presented.

VILKNER T ET AL: "MICRO TOTAL ANALYSIS SYSTEMS. RECENT DEVELOPMENTS" ANALYTICAL CHEMISTRY, AMERICAN CHEMICAL SOCIETY. COLUMBUS, US, vol. 76, no. 12, 15 June 2004, pages 3373-3385 represents a further review of micro total analysis systems, presenting recent developments of microfluidic devices, in principle, giving supplementary information to document REYES D R ET AL and AUROUX P-A ET AL.

COX J D ET AL: "Surface passivation of a microfluidic device to glial cell adhesion: a comparison of hydrophobic and hydrophilic SAM coatings" BIOMATERIALS, ELSEVIER SCIENCE PUBLISHERS BV., BARKING, GB, vol. 23, no. 3, February 2002, pages 929-935 describes the effect of surface passivation of a microfluidic device to glial cell adhesion. The effects of hydrophobic octadecyltrimethoxysilane and hydrophilic N-(triethoxysilylpropyl)-O-polyethylene oxide urethane as coatings for silicon dioxide wafers were examined as surface passivation.

LIPOWSKI AND SACKMANN: "Handbook of Biological Physiscs" 1995, ELSEVIER SCIENCE B.V , page 755 - page 802 is a copy of chapter 16, "Adhesion of Cells" of the "Handbook of Biological Physics" and teaches the principles of cell adhesion, for example the interactions between cells and artificial surfaces.

L. Barbe, These pour obtenir le grade de docteur a L'Université Paris 7, U.F.R de physique. "Mecanisme d'adherence des leucocytes aux fibres synthétiques. Application à la filtration du sang", 14.12.2001 relates to leukocyte adhesion during blood filtration in a mini-filter and a flow chamber. In chapter 1, item 4.2.3, leukocyte adhesion is discussed subject to the surface tension of the filter material. Polyethylene terephthalate (PET) fibers are considered the most efficient for blood filtration resulting in the best leukocyte cell adhesion.

Wang Z. et al.; Lap Chip; 2004, 4, 372-736 describes a microchip flow cytometer having all optical elements integrated in one layer of the microchip polymer, here, SU-8. Ikada; Biomaterials; 1994, 15(10), pages 725-736 refers to surface modification of polymers for medical applications. According to pages 734 and 735, macrophage adhesion on surfaces is enhanced if using polystyrene, PET or nylon.

Chung et al., Microsystems Technologies, 2004, Vol. 10, pp. 81-88 relates to multi-height microstructures in a PDMS lab-on-a-chip. In this paper, several fabrication schemes are described and an overview is given over the technologies used for PDMS replica molding and channel closing with oxygen plasma treatment. In the final section, fabrication results of multi-height structures for lab-on-a-chip are presented, such as the systems for platelet extraction from whole blood or erythrocyte filtering, respectively, consisting of microchannels having filter structures comprising pillars.

US 2003/0049563 relates to an apparatus for fractionating a sample into various sorts of microstructures different in size, e.g. cells, DNA and/or proteins. The apparatus comprises a fractionating unit including a region for permitting the sample to be migrated and at least one colony of microbodies serving as obstacles against the migration of the microstructures. The colony of microbodies defines a labyrinth for trapping small-sized microstructures so that a remaining area serves as a path for large-sized microstructures.

If the concentration of bacteria or viruses is very low at an early stage of purification of nucleic acid from the cells or the viruses, the cells or the viruses must be enriched. Since the volume of samples used in a miniaturized chip such as a Lab-on-a-chip is generally very small, in that case the cell enrichment is especially necessary.

The inventors of the present invention has discovered, while conducting research on ways to separate cells or viruses, that the cells or viruses can be rapidly and efficiently separated by using a hydrophobic solid support having a specific water contact angle and has completed the present invention.

### SUMMARY OF THE INVENTION

The present invention provides a cell or virus separation method using a hydrophobic solid support, including contacting a solution containing cells or viruses with a hydrophobic solid support having a water contact angle between 70 and 90 degrees, wherein the hydrophobic solid support has a pillar structure, wherein the contacting is carried out in a fluidic state and the pH of the solution containing cells or viruses is in the range of 2.5 to 4, wherein in the contacting, salt is further added and the concentration of the salt has a range of 0.01 to 2 M, and wherein the cells are bacteria.

### BRIEF DESCRIPTION OF THE DRAWINGS

The above and other features and advantages of the present invention will become more apparent by describing in detail exemplary embodiments thereof with reference to the attached drawings in which:
FIG. 1 is a graph illustrating the number of *E*. *coli* cells captured by the solid supports having various water contact angles at pH 4 and 7;
FIG. 2 is microscopic images of a hydrophilic alumina substrate having a water contact angle of 5 degrees or less, by which no *E*. *coli* cells are captured at pH 4 and 7;
FIG. 3 is microscopic images of *E*. *coli* cells captured by the solid support of the present invention in a flow control system;
FIG. 4 is microscopic images of *Pseudomonas putida* and *E*. *coli* BL21 cells, which are gram negative bacteria, captured by the solid support of the present invention in a flow control system;
FIG. 5 is microscopic images of *Streptococcus mutans* and *Staphylococcus epidermidis* cells, which are gram positive bacteria, captured by the solid support of the present invention in a flow control system;
FIG. 6 is microscopic images of *Staphylococcus epidermidis* as gram positive bacteria, illustrating that the number of bacteria captured by the solid support of the present invention is increased due to the additive such as alcohol;
FIG. 7 is microscopic images of *E*. *coli* BL21 as gram negative bacteria, illustrating that the number of cells captured by the solid support of the present invention is increased due to an additive such as alcohol;
FIG. 8 is top views of designed diamond-shaped pillars and brick-shaped pillar arrangements;
FIG. 9 is SEM images of chips having the diamond-shaped pillar and the brick-shaped pillar arrangements manufactured using a self assembled monolayer (SAM) coating method on a silicon substrate;
FIG. 10 is a SEM image of a chip having a diamond-shaped pillar arrangement manufactured using SU-8; and
FIG. 11 is microscopic images of gram positive and negative bacteria captured by a chip having a pillar structure, which is the hydrophobic solid support having a three-dimensional structure of the present invention.

### DETAILED DESCRIPTION OF THE INVENTION

According to an aspect of the present invention, there is provided a method of separating cells or viruses using a hydrophobic solid support, including contacting a solution containing cells or viruses with a hydrophobic solid support having a water contact angle between 70 and 90 degrees, wherein the hydrophobic solud support has a pillar structure, wherein the contacting is carried out in a fluidic state and the pH of the solution containing cells or viruses is in the range of 2.5 to 4, wherein in the contacting, salt is further added and the concentration of the salt has a range of 0.01 to 2 M, and wherein the cells are bacteria.

In this method, an additive capable of inducing cell aggregation or precipitation may be added to increase the extent of capturing of target cells.

One of the methods of cell capture and separation applicable to a miniaturized chip is to use a solid substrate. When a solid substrate is used, it is advantageous in an implementation of a LOC (lab-on-a-chip) and cells can be captured by a physical interaction between cells and the solid substrate. The interaction includes an electrostatic adsorption and a hydrophobic interaction. The former can separate cells using a material such as alumina which has an electrostatic positive charge because generally the surface of cell is negatively charged. The latter is a cell attachment resulting from physical interaction originated from the surface free energy between cells and a solid support, which is governed by surface thermodynamics. Because the hydrophobicity of their surfaces of a cell and solid support might be dependent on the pH of surrounding environment, the pH may be an important factor. A typical method of identifying the hydrophobicity of the solid support surface is to measure a water contact angle. As the water contact angle of the solid surface increases, the hydrophobicity increases.

When a solution including cells or viruses is in contact with a hydrophobic solid support having a water contact angle between 70 and 90 degrees, cells or viruses bind to the solid support due to hydrophobic interaction with the hydrophobic solid support. Cells or viruses rarely bind to a hydrophilic solid support as demonstrated in Examples below. In addition, when the water contact angle of a hydrophobic solid support does not lie within the above-described range, the degree of binding of cells or viruses to the hydrophobic solid support decreases.

The contact of cells or viruses with the hydrophobic solid support carried out at a range of pH 2.5 to 4. This is because the binding efficiency of cells or viruses to the hydrophobic solid support is significantly reduced when the pH of a solution including cells or viruses does not lie within the above-described range.

In an embodiment of the present invention, the method may further include a washing step of the hydrophobic solid support having cells or viruses bound thereto. When the hydrophobic solid support having cells or viruses bound thereto is washed with a washing solution such as a phosphate buffered solution, things that do not bind to the hydrophobic solid support are removed and cells or viruses bound are remained. Thus, cells or viruses can be selectively separated from a mixture containing the cells or viruses. Thus cells or viruses can be concentrated.

In the method, the solution containing cells or viruses can be saliva, blood, urine, buffer or a combination thereof, but is not limited thereto.

In the method, the contact of a solution containing cells or viruses with a hydrophobic solid support carried out in a flowing system. In the flow control system, the hydrophobic solid support has a three dimensional structures, namely as a pillar. Those 3-D structures enable to capture more cells or viruses by increasing the collision rate of cells or viruses with the hydrophobic solid support.

In the method, the aspect ratio of a pillar post may be a range of 1:1 to 20:1. The aspect ratio refers to a ratio of the height to the width of a pillar post. When the aspect ratio does not lie within the above-described range, the binding efficiency of cells or viruses to the hydrophobic solid support decreases.

In the method, the ratio of the height of a pillar post (H) to the distances between pillar posts may be a range of 1:1 to 25:1. When the ratio is less than 1:1, the efficiency of capturing cells or viruses decreases.

In the method, the distance (D) between pillars may be a range of 5 to 100 µm.

When the distance (D) between pillars is less than 5 µm, it is difficult to fabricate them on a solid support such as a silicon surface. When the distance (D) between pillars is greater than 100 µm, the number of pillars on the same area decreases, and thus the efficiency of capturing cells or viruses decreases.

In the method, the hydrophobic solid support may take one of the forms such as substrates, particles, sheets, gels, filters, membranes, fibers, capillaries, tubes, plates or wells. The hydrophobic solid support may be any support capable of capturing cells or viruses, but should not be dissolved in the solution containing cells or viruses.

Conveniently, the support may be made of glass, silica, latex or a polymeric material. Preferred are materials presenting a high surface area for binding of the cells. Such supports will generally have an irregular surface and may be, for example, porous or particulate. The hydrophobic solid support may be made of a material such as, tridecafluorotetrahydrooctyl trimethoxysilane (DTS). The above-listed materials are represented by formulae:

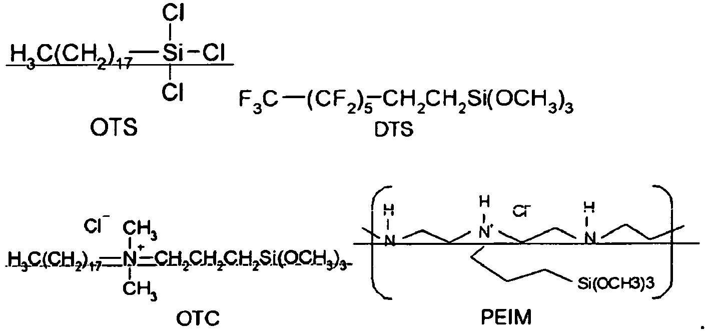

This materials can provide the solid support with a desired hydrophobicity and is not particularly defined as long as it can capture cells or viruses.

In the method, the cells are bacteria or viruses including, bacteriophages, plant viruses and animal viruses. Both gram positive and negative bacteria can bind to the hydrophobic solid support. By adding a material capable of inducing cell aggregation and precipitation, such as ethanol, the binding efficiency of bacteria to the hydrophobic solid support can be further increased.

In the method, an alcoholic material capable of inducing cell aggregation and precipitation may be added in the contacting a solution containing cells or viruses with a hydrophobic solid support. The binding efficiency of cells on the solid support can be increased by aggregating cells or precipitating cells on the solid support using the additive. For example, an increase in the efficiency can be achieved by contacting cells with the support in the presence of alcohol and salt. The use of alcohol and salt in separation and purification procedures such as precipitation is commonplace and any suitable alcohol or salt used in such procedures, may be used according to the present invention. Thus, the alcohol may be any alkanol, and lower alkanols such as isopropanol and ethanol are suitable. Other suitable alcohols include methanol and n-butanol.

The salt may be provided by any convenient source, for example, sodium or potassium chloride or acetate, or ammonium acetate. Appropriate concentrations of alcohol and salt may be determined according to the system and reagents used. Generally, 0.5 to 3 volumes of alcohol, for example 1 volume, to the sample is suitable. Conveniently, the alcohol may be used at concentrations of 50-100% (w/v). The salt concentration has a range of 0.01 to 2.0 M, and conveniently the salt may be included at the above concentrations in the alcohol solution. Thus, a so-called "cell-binding buffer" may be used containing the alcohol and salt at the desired concentrations.

The use of alcohol as a precipitant for the cells according to the invention is advantageous for use of the method in clinical diagnostic procedures, since the use of alcohol to conserve clinical samples is common. Thus, patient samples may simply be added to an alcohol-containing cell-binding buffer, whereby the samples are conserved.

Other precipitants may be used, for example, polyethylene glycols (PEGs) or other high molecular weight polymers with similar properties, either alone or in combination with salt and/or alcohol. The concentrations of such polymers may vary depending upon the precise system, for example, polymer and cell type, but generally concentrations from 1 to 50% (w/v), for example, 2-30% may be used.

Cells with phagocytic activity may be captured by their ability to bind or swallow a particulate solid phase, for example, beads, and thereby can readily be separated. In this case, the cell-containing sample needs simply to be contacted or incubated with the solid phase under appropriate conditions. This kind of cell capture is not dependent on specific binding.

Finally, as mentioned above, cell-binding to solid supports having charged, hydrophobic surfaces is achieved by using buffers, often combination with salt, to achieve pH conditions appropriate for binding. The precise buffers and conditions will vary depending on the type of cell, solid support etc.

Various components are mixed and simply allowed to stand for a suitable interval of time to allow the cells to bind to the support. The support may then be removed from the solution by any convenient means, which will depend, of course, on the nature of the support, and includes all forms of withdrawing the support away from the sample supernatant, or vice versa, for example, centrifugation, pipetting, etc.

The conditions during this process are not critical, and, for example, simply to mix the sample with the "cell-binding buffer" in the presence of a solid phase, and allow it to stand at room temperature, for example, for 5 to 30 minutes, for example, 20 minutes before separating. As mentioned above, the reaction time is critical and as little as 5 minutes may be often enough. However, longer periods may be used, for example, 20 minutes to 3 hours, or even overnight. Mixing can be done by any convenient means including stirring or vortexing. Also, if desired, higher or lower temperatures may be used.

The present invention will now be described in greater detail with reference to the following examples. The following examples are for illustrative purposes only and are not intended to limit the scope of the invention.

### Example 1: Separation of Escherichia coli cells using hydrophobic solid substrate of the present invention

To find out whether bacterial cells can efficiently be separated by the method of the present invention, *Escherichia coli (E. coli)* HB101 strain as a gram negative cell was used. A substrate used in the experiment was a substrate made of octadecyltrichlorosilane (OTS), tridecafluorotetrahydrooctyl trimethoxysilane (DTS), octadecyldimethyl(3-trimethoxysilyl propyl)ammonium chloride (OTC), or polyethyleneiminetrimethoxysilane (PEIM).

### 1) Cell culture

*E. coli* cells were cultured according to a common procedure at 37°C till exponential phase overnight.

### 2) Preparation of cell-containing solution

*E. coli* cells cultured overnight were centrifuged at x 800 g for 5 minutes to precipitate cells. Then, the cells were washed three times with 5 ml of 1 ×PBS (phosphate buffered saline, pH 7.4, Invitrogen corporation). Washing was carried out by repeating the process including resuspending *E. coli* cells precipitated in 1 ×PBS and centrifuging the resuspended *E. coli* cells at x 800 g for 5 minutes. The washed *E. coli* cells were suspended in 5 ml of a 0.1 M phosphate buffer (pH 4) and 5 ml of a 0.1 M phosphate buffer (pH 7), respectively. The initial quantity of the suspended *E. coli* cells was obtained by measuring the optical density (OD) thereof using a UV spectrophotometer. The quantity of the initial *E. coli* cells, in 0.1M phosphate buffer (pH 4), was 0.475 in OD at 600 nm, which indicated 1.43E+07 cells for 60 µℓ and, in 0.1 M phosphate buffer (pH 7), was 0.454, which was presumed to be 1.36E+07 cells. Generally, 1 in OD at 600nm indicates the concentration of 5.0E+08 cells/ml for *E. coli.*

### 3) Manufacturing a solid substrate

A solid substrate was manufactured using a self assembled monolayer (SAM) method. The detailed manufacturing method was as follows.

### (1) Washing of glass

Glasses were immersed in a piranah solution for 2 hours or more. Then, the glasses were spin dried one by one.

### (2) Preparation of coating solution

OTS and DTS were mixed with toluene such that the final concentration of each mixture was 100 mM. Then, OTS and PEIM were mixed with EtOH and stirred for 1 hour.

### (3) Immersion of glass

The glass prepared above (1) was immersed in the solution prepared above (2) for 4 hours.

### (4) Washing of glass

The immersed glass was washed three times with EtOH for each 10 minutes, and then dried in vacuum.

### (5) Incubation

The dried glass was incubated at 120°C for 1 hour.

### 4) Cell binding

60 µℓ Patch was attached to the solid substrate manufactured above, and then 60µℓ of the cell suspension was applied to the substrate. The substrate was incubated at room temperature for 5 minutes, and then washed with 30 ml of a 0.1 M sodium phosphate buffer (pH 4) and 30 ml of a 0.1 M sodium phosphate buffer (pH 7) for each 10 minutes. The washing was carried out in the same manner as described above.

### 5) Measurement

To stain *E. coli* cells bound to the solid substrate, the *E. coli* cells were stained with a gram stain solution for *E. coli* cell known in the art. First, a crystal violet solution was sufficiently applied to the region to which cells were bound. After 1 minute, the region was washed with flowing water. Then, a gram iodine solution, a gram decolorizer, and a gram safranin solution were treated in the same manner to complete the gram stain. After the gram stain, the solid substrate was air dried at room temperature. Then, an image of 3 points was captured using an optical microscope at a magnification of ×2000 to determine the number of cells captured per unit area.

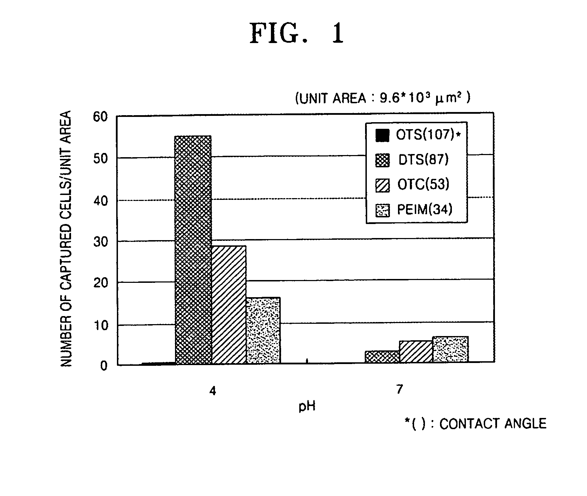

FIG. 1 is a graph illustrating the number of *E. coli* cells captured by the solid support having various water contact angle at pH 4 and 7. Unit area is 9.6×10³ µm² and numerals in parentheses indicates a water contact angle. The left panel indicates the number of cells captured at pH 4 and the right panel indicates the number of cells captured at pH 7. Referring to FIG. 1, when the buffer having a pH of 7 is used, the *E. coli* cells rarely bound to the solid support compared to when the buffer having a pH of 4 is used. Thus, it can be seen that pH is very important when contacting cells or viruses with the solid substrate.

Further, it can be seen that the solid substrate made of DTS among 4 types of solid substrate has the highest cell binding efficiency. The solid substrate made of DTS has a water contact angle of 87 degrees and has higher cell capture efficiency than other solid substrates having different water contact angles, which indicates that the water contact angle is very important for the cell capture efficiency.

### Comparative Example 1: Separation of Escherichia coli cells using an alumina substrate

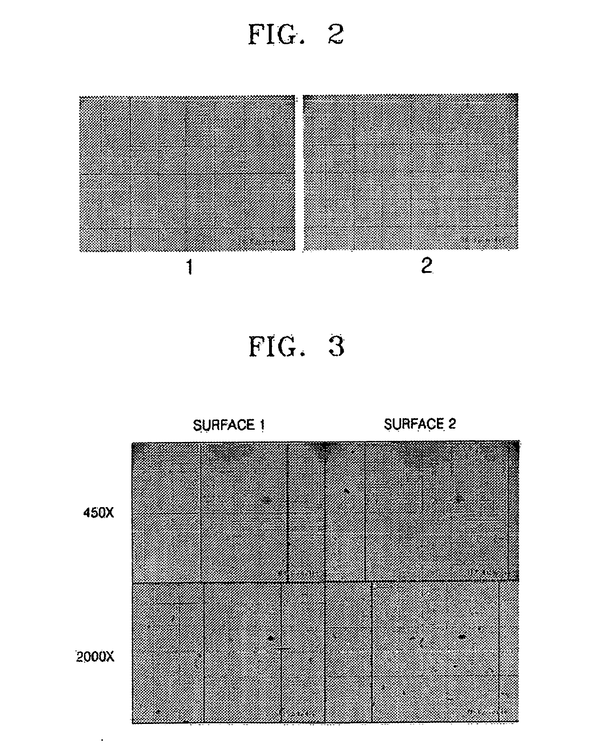

The experiment was carried out in the same manner as in Example 1 except that an alumina substrate having a water contact angle of 14.6 degrees was used instead of the solid substrate used in Example 1. FIG. 2 is microscopic images of a hydrophilic alumina substrate, by which no *E. coli* cells were captured at pH 4 and pH 7. In FIG. 2, Panel 1 is an image of the alumina substrate at pH 4 and Panel 2 is an image of alumina substrate at pH 7. Referring to FIG. 2, *E. coli* cells rarely bound to the substrate at both pH 4 and 7. That is, *E. coli* cells rarely bound to the hydrophilic alumina having a water contact angle of 30 degrees or less, and thus it can be seen that the water contact angle is an important factor in the binding ability of *E. coli* cells to a substrate.

### Example 2: Separation of Escherichia coli cells under flow control

Unlike Example 1, to investigate of the binding ability of *E. coli* to the substrate of the present invention in a flow control system, a charge reversible surface (CRS) having a water contact angle of 75 degrees (Korean Patent Application No. 2005-0030286) was used as a surface of a solid substrate. A syringe pump (HARVARD, PHD2000), a flow rate of 0.3 cm/sec (900µℓ/min), total surface area of 5 mm x17.3 mm, a cross-sectional area of 5 mm x 1.05 mm=5mm² at an aspect ratio of 5:1 or more, 200 µℓ of *E. coli* HB101 as bacterial cell, and pH 4 (bacterial concentration 1.0E+05 cells/µℓ) were used. At each flow rate, 200 µℓ of bacterial cells were allowed to once pass through the surface area. The substrate was washed while once flowing 1 ml of the sodium phosphate buffer (pH 4, 0.1 M) at flow rate of 900 µℓ/min. The subsequent procedures were carried out as in Example 1.

FIG. 3 is microscopic images of *E. coli* cells captured by the solid support of the present invention in a flow control system. Surfaces 1 and 2 are the results obtained by duplicate experiments. Referring to FIG. 3, *E. coli* cells bound to the solid substrate are not distinguished at a magnification of ×450, but rod-shaped *E. coli* cells are observed at a magnification of ×2000. Thus, similarly to a fixed system, *E. coli* cells also efficiently bind to the substrate of the present invention in the flow control system.

### Example 3: Separation of four different types of bacterial cells under flow control

The experiment was carried out in the same manner as in Example 2 except that *E. coli* BL21 and *Pseudomonas Putida* as gram negative bacteria and *Streptococcus mutans* and *Staphylococcus epidermidis* as gram positive bacteria were used instead of *E. coli* HB101.

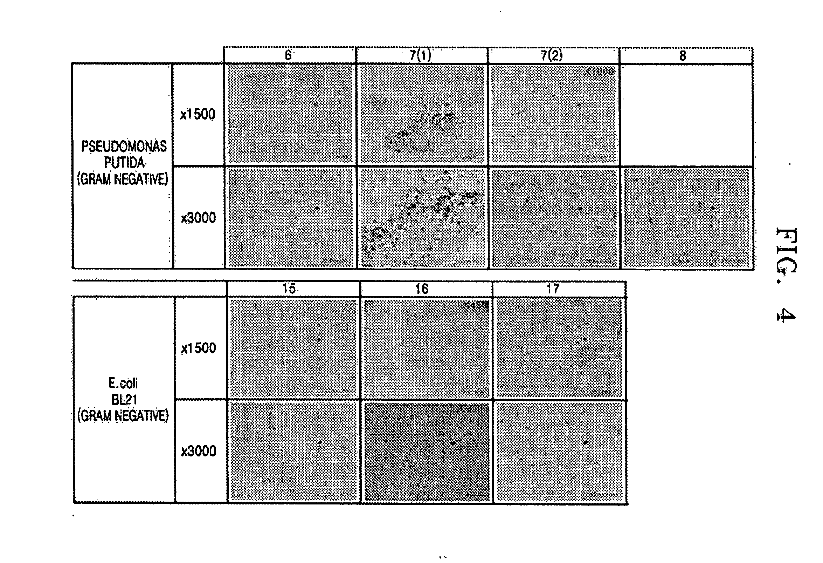

FIG. 4 is microscopic images of *Pseudomonas putida* and *E. coli* BL21 cells, which are gram negative bacteria, captured by the solid support of the present invention in a flow control system. Nos. 6, 7(1), 7(2), 8, 15, 16, and 17 represent Sample Nos. Referring to FIG. 4, *Pseudomonas putida* and *E. coli* BL21 cells relatively uniformly attached to the surface of the solid substrate. In addition, part of cell aggregation as in Sample 7(1) is observed. Thus, *Pseudomonas putida* and *E. coli* BL21 cells efficiently bind to the solid substrate of the present invention in the flow control system.

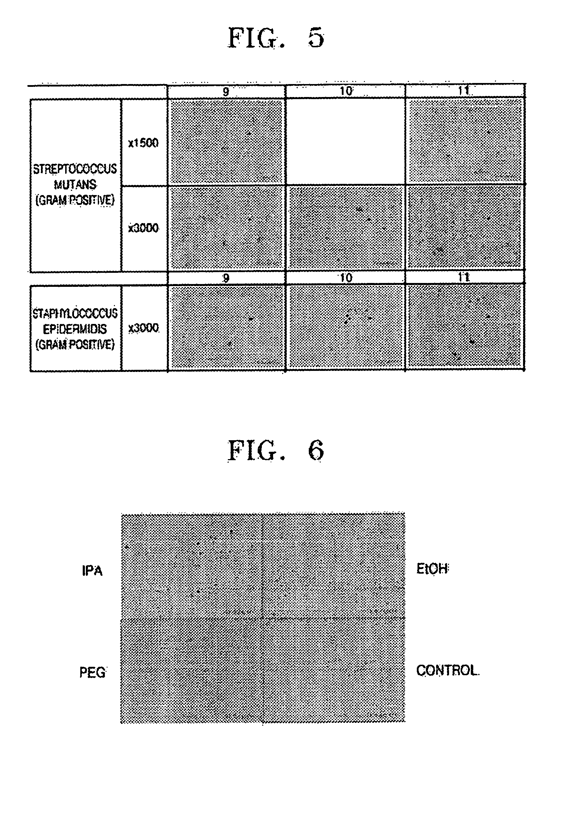

FIG. 5 is microscopic images of *Streptococcus mutans* and *Staphylococcus epidermidis* cells, which are gram positive bacteria, captured by the solid support of the present invention in a flow control system. Nos. 9, 10 and 11 represent Sample Nos. of *Streptococcus mutans* and Nos. 12, 13 and 14 represent Sample Nos. of *Staphylococcus epidermidis.* Referring to FIG. 5, *Streptococcus mutans* and *Staphylococcus epidermidis,* which are gram positive bacteria, bind to the surface of the solid substrate of the present invention, but the binding efficiency thereof is much lower than that of gram negative bacteria shown in FIG. 4.

The number of *Pseudomonas putida* and *E. coli* BL21 cells, that are gram negative bacteria, attached to the solid substrate of the present invention is summarized in the following Table.

| Bacterial cell | Number of attached cells | Cells/µm² | Cells/86.5 mm² |
|---|---|---|---|
| *Pseudomonas putida* | 10.00 | 0.00208 | 1.80E+05 |
| *E. coli* BL21 | 8.40 | 0.00175 | 1.51 E+05 |

### Example 4: Separation of cells using an additive

To investigate the binding ability of cells to the substrate of the present invention according to the addition of alcohol and/or salt in a flow control system, ethyltrimethoxysilane having a water contact angle of 70 degrees was used as a surface of the solid substrate. A syringe pump (HARVARD, PHD2000), a flow rate of 0.6 cm/sec (900 µℓ/min), total surface area of 5 mm ×17.3 mm, a cross-sectional area of 5 mm×0.5 mm=2.5mm² at an aspect ratio of 10:1 or more, and 200 µℓ of *E. coli* BL21 and 200 µℓ of *Staphylococcus epidermidis* (2.0E+07 cells) as bacterial cell were used.

PEG 10000 (w/v 20%) ("PEG" in FIG. 6) in a 0.1 M phosphate buffer (pH 4), EtOH (v/v 50%) ("EtOH" in FIG. 6) in a 0.1 M phosphate buffer (pH 4), and IPA (v/v 25%) ("IPA" in FIG. 6) in 3M NaCl (pH 4) were added to a cell suspension medium as additives and a 0.1 M phosphate buffer (pH 4) was used as control. At each flow rate, 200 µℓ of bacterial cells were allowed to once pass through the surface area. The substrate was washed while once flowing 1 ml of the sodium phosphate buffer (pH 4) at flow rate of 900 µℓ/min. The subsequent procedures were carried out as in Example 1.

FIG. 6 is microscopic images of *Staphylococcus epidermidis* as gram positive bacteria, illustrating that the number of bacteria captured by the solid support of the present invention is increased due to the additive such as alcohol. These images are taken at a magnification of ×1000. Referring to FIG. 6, *Staphylococcus epidermidis* rarely bound to the substrate when the 0.1 M phosphate buffer (pH 4) was used as control. Also, even when PEG was added, cells rarely bound to the substrate. However, in the case of IPA and EtOH, a substantial amount of cells bound to the substrate. Thus, the number of gram positive cells bound to the hydrophobic solid substrate of the present invention is significantly small when alcohol and/or salt is not added, but is significantly increased when alcohol and/or salt is added.

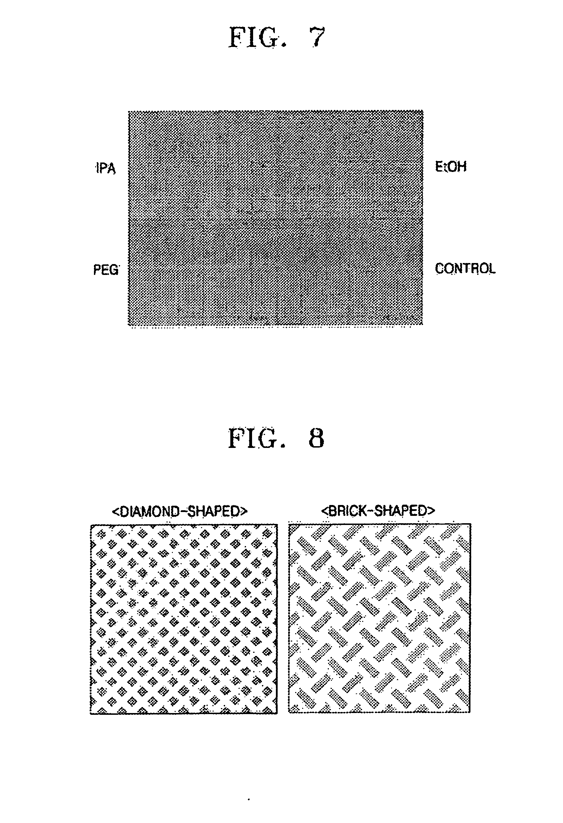

FIG. 7 is microscopic images of *E. coli* BL21 as gram negative bacteria, illustrating that the number of cells captured by the solid support of the present invention is increased due to an additive such as alcohol. These images were taken at a magnification of ×1000 and the unit area was 6×10⁴ µm². The number of *E.* coli BL21 cells bound to the unit area (6×10⁴ µm²) was 33 in control, 23 in PEG, 123 in IPA, and 145 in EtOH. Referring to FIG. 7, although *E. coli* BL21 as gram negative bacteria bound to the substrate even when 0.1 M phosphate buffer (pH 4) was used as control and when PEG was added, more cells bound to the substrate when IPA and EtOH were added. Thus, gram negative cells also more easily bind to the hydrophobic solid substrate of the present invention when alcohol and/or salt is added than when alcohol and/or salt is not added.

### Example 5: Manufacturing microchip having hydrophobic solid support of a three-dimensional structure

To investigate the binding ability of *E. coli* to a having a solid support of a three-dimensional structure unlike Example 2 using a planar solid substrate of a two-dimensional structure, two types of microchips were manufactured.

A silicon-based chip (Si/SiO₂/SAM) was manufactured as follows:

### (1) Wafer cleaning

A wafer was treated in a Piranah solution (H₂SO₄:H₂O₂=3:1, 120°C) for 15 minutes, and then washed with flowing water and dried.

### (2) HMDS coating

After applying 5 ml of HMDS to the cleaned wafer using a spin coater, coating was carried out at 500 rpm for 5 seconds and at 4000 rpm for 40 seconds, and then baking was carried out on a hot plate at 120°C for 2 minutes.

### (3) PR coating

After applying 5 ml of photoresist (GXR 601) to the wafer, coating was carried out at 500 rpm for 5 seconds and at 4000 rpm for 40 seconds.

### (4) Soft baking

Baking was carried out using a hot plate at 95°C for 2 minutes.

### (5) UV exposure

A mask for manufacturing a pillar was mounted on a UV aligner (I-line), and then 250 mJ was irradiated.

### (6) Development

Development was carried out using a MIF 300 developing apparatus.

### (7) Hard baking

The developed wafer was hard baked at 115°C for 2 minutes.

### (8) Deep RIE

### An Si etching process of 100 µm was carried out using STS ICP-RIE apparatus.

### (9) Ashing

The photoresist was ashed using an asher.

### (10) PR strip

To remove residue PR, the wafer was treated with a Piranah solution for 15 minutes, rinsed, and dried.

### (11) HF treatment

The wafer was treated with diluted HF for 1 minute to remove native oxide.

### (12) Si oxidation

Thermal wet oxidation was carried out using water vapor to grow SiO₂ to a thickness of 1000 Å.

### (13) Wafer cleaning

The wafer was treated with a Piranah solution for 15 minutes, and then rinsed and dried.

### (14) SAM coating

The wafer was immersed in a 200 mM octadecyldimethyl(3-trimethoxysilyl propyl) ammonium chloride (OTC) in ethanol for 1 hour, and then washed three times with ethanol and dried. A baking process was carried out at 110°C for 40 minutes.

Next, a SU-8 chip was manufactured as follows:

### (1) Wafer cleaning

The wafer was treated with a Piranah solution for 15 minutes and with diluted HF for 3 minutes, and then rinsed and dried. The wafer was then treated on a hot plate at 200°C for 20 minutes.

### (2) SU-8 coating

8 ml of SU8 2100 (Microchem) was poured to the wafer, and then spin coating was carried out at 500 rpm for 30 seconds and 3000 rpm for 30 seconds.

### (3) Soft baking

The SU8-coated wafer was treated on a hot plate at 65°C for 5 minutes and at 95°C for 20 minutes.

### (4) UV exposure

A mask for manufacturing a pillar was mounted on a UV aligner (I-line), and then 320 mJ was irradiated.

### (5) Post exposure baking

The wafer was treated at 65°C for 1 minute and at 95°C for 20 minutes.

### (6) Development

Development was carried out using a SU8 developing apparatus. (Microchem) for 30 minutes.

### (7) Washing

The wafer was simply washed with 2-propanol and dried with nitrogen.

The three-dimensional structure of the chip was a pillar structure, a chamber volume was 10µℓ, the pillar height was 100 µm, and 4 types of diamond-shaped pillars and 2 types of a brick-shaped pillars were manufactured as follows.

| No. of silicon-based chip | Type of pillar | Height of pillar (µm) | Size of pillar (µm) | Distance between pillars (µm) | Aspect ratio | Size of chip (mm) | Volume of chamber (µℓ) |
|---|---|---|---|---|---|---|---|
| 1 | Diamond-shaped | 100 | 25×25 | 25 | 4 | 33.89×9.89 | 10 |
| 2 | Diamond-shaped | 100 | 50×50 | 50 | 2 | 33.89×9.89 | 10 |
| 3 | Diamond-shaped | 100 | 25×25 | 8 | 4 | 33.89×9.89 | 10 |
| 4 | Diamond-shaped | 100 | 50×50 | 17 | 2 | 33.89×9.89 | 10 |
| 9 | Brick-shaped | 100 | 25×75 | 25 | 4 | 33.89×9.89 | 10 |
| 10 | Brick-shaped | 100 | 25×50 | 12.5 | 4 | 33.89×9.89 | 10 |

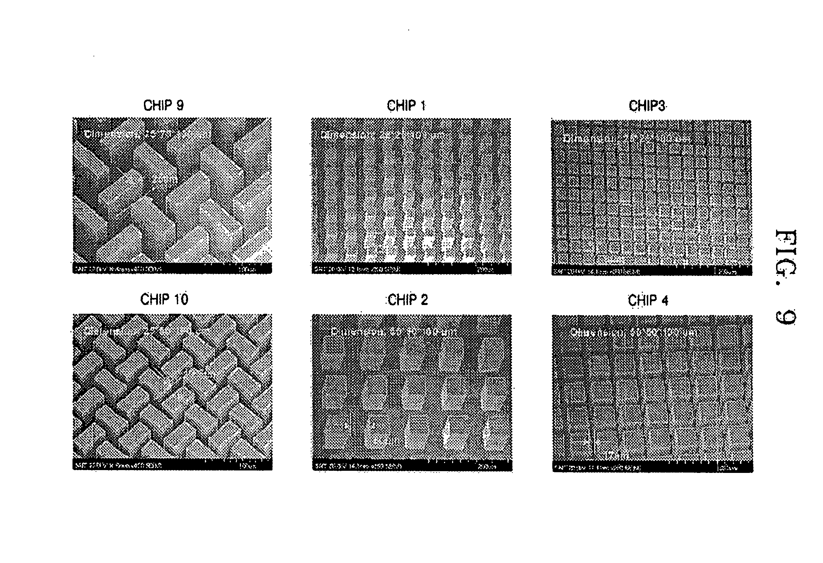

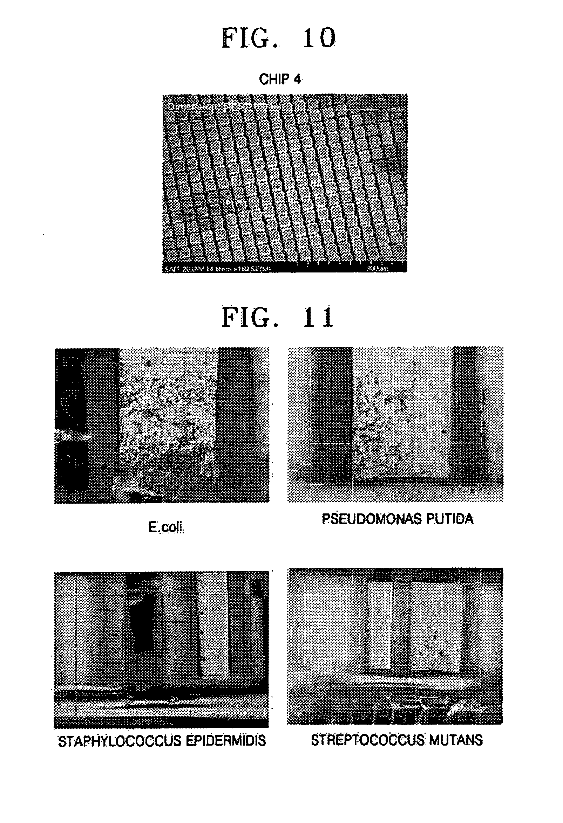

FIG. 8 is top plan views of designed diamond-shaped pillars and brick-shaped pillar arrangements. Such chips were manufactured and observed with a scanning electron microscope (SEM). FIG. 9 is SEM images of chips having diamond-shaped pillar and brick-shaped pillar arrangements manufactured using a SAM method on a silicon substrate. Referring to FIG. 9, it can be seen that diamond-shaped chips and brick-shaped chips having desired dimensions were manufactured. FIG. 10 is a SEM image of a chip having a diamond-shaped pillar arrangement manufactured using SU-8. Referring to FIG. 10, it can be seen that a diamond-shaped chip having a desired dimension was manufactured.

### Example 6: Separation of E. coli cells using three-dimensional microchip

Unlike Example 2 using a planar solid substrate, the three-dimensional microchip manufactured in Example 5 was used to investigate the binding ability of *E. coli* to the microchip of the present invention in a flow control system. *E. coli* BL21 of 1.0E+08 cells/ml was used and the flow rate was 400 µℓ/min. The number of *E. coli* cells bound to the microchip was determined using a method of measuring the number of colony. The subsequent procedures were carried out in the same manner as in Example 2.

The capture efficiency for *E. coli* cells according to the type of chip is shown in Table 1. "Plane" indicates the case where the planar solid substrate of Example 2 was used.

**Table 1**

| Type of chip | Pillar size (µm) | Distance between pillars (µm) | Total number of pillar | Increase in surface area | Ratio of surface to volume | Capture efficiency (%) |
|---|---|---|---|---|---|---|
| Chip 1 | 25×25 | 25 | 48422 | 6.6 | 0.063 | 91.69±7.1 |
| Chip 2 | 50×50 | 50 | 12106 | 3.8 | 0.037 | 24.7 |
| Chip 3 | 25×25 | 8 | 181764 | 21.9 | 0.226 | 97.0±2.5 |
| Chip 4 | 50×50 | 17 | 42613 | 10.8 | 0.111 | 90.1±3.7 |
| Chip 9 | 25×75 | 25 | 23706 | 6.6 | 0.063 | 52.8±7.6 |
| Chip 10 | 25×50 | 12.5 | 64155 | 13.1 | 0.140 | 83.3±12.7 |
| Plane | - | - | - | - | 0.002 | ~1 |

As is apparent from the above Table, Chip 3 having a greatest increase in surface area of pillar had highest *E. coli* capture efficiency. This indicates that several ten times higher *E. coli* capture efficiency than when a planar surface is used (capture efficiency of about 1 %) was achieved. Thus, the chip having a pillar structure of the present invention increases opportunity to contact with *E. coli,* and thus can more efficiently capture *E. coli* than a planar structure.

### Example 7: Separation of cells using three-dimensional microchip according to a low concentration of bacterial cells and the kind of bacterial cells

In the present Example, unlike Example 6, a low concentration of *E. coli* cell and other bacterial cells were used to investigate of cell capture efficiency of the three-dimensional microchip of the present invention. The experiment was carried out in the same manner as in Example 6, except that *E. coli* BL21 of 1.0E+03 cells/ml and 1.0E+05 cells/ml were used as *E. coli* and *Pseudomonas putida, Staphylococcus epidermidis* and *Streptococcus mutans* of each 1.0E +08 cells/ml were used as other bacterial cells, and Chip 10 was used.

The cell capture efficiency according to the kind of bacterial cells is shown in Table 2.

**Table 2**

| Type of cell | Cell concentration (cells/ml) | Capture efficiency (%) |
|---|---|---|
| *E. coli* | 1×10³ | 56.2±14 |
| | 1×10⁵ | 56.2±6.3 |
| *Pseudomonas putida* | 1×10⁸ | - |
| *Staphylococcus epidermidis* | 1×10⁸ | 76.2±11.5 |
| *Streptococcus mutans* | 1×10⁸ | 79.6±2.7 |

In the Table 2, the capture efficiency for *Pseudomonas putida* could not determined since the cell did not grow at pH 4 which is a condition for binding of cells, but capture of the cell could be identified through a microscopic image analysis.

As is apparent from the Table 2, the capture efficiency for *E. coli* BL21 is high at a low concentration and the capture efficiency for bacterial cells other than *E. coli* are also very high. Thus, the chip having a pillar structure of the present invention can efficiently capture most bacterial cells.

To observe bacteria captured by the chip of the present invention, the captured bacterial cells were stained according to a common staining procedure and observed through an electron microscope. FIG. 11 is microscopic images of gram positive and negative bacteria captured by a chip having a pillar structure, which is the hydrophobic solid support having a three-dimensional structure of the present invention. Referring to FIG. 11, it can be seen that bacterial cells were efficiently captured by pillars.

### Example 8: Separation of E. coli cells included in a real sample using three-dimensional microchip

In the present Example, a suspension of a saliva sample and bacterial cells was prepared to investigate the capture efficiency for bacterial cells included in a real sample. *E. coli* BL21 stained with a SYTO-9 fluoresecent dye was used as a bacterial cell. The saliva sample was pre-treated with 0.01 M dithiothreitol (DTT) for 15 minutes or more and centrifuged, followed by resuspending the resultant in a 0.1 M sodium phosphate buffer (pH 4) to dilute to a 1/4 of the concentration of the initial saliva sample. The pre-treated saliva solution was suspended with the SYTO-9 stained bacteria. The flow rate was 400 µℓ/min and capture of bacteria was determined using a fluoremeter. The subsequent procedures were carried out in the same manner as in Example 6.

The capture efficiency for *E. coli* cells included in the real sample is shown in Table 3.

**Table 3**

| Type of chip | Cell concentration (cells/ml) | Capture efficiency (%) |
|---|---|---|
| Chip 4 (Si/SiO₂/SAM chip) | 5×10⁶ | 99.6±0.1 |
| SU-8 chip (the same design as Chip 4, except for material | 2×10⁷ | 98.4±0.3 |

As is apparent from Table 3, both the Si/SiO₂/SAM chip and the SU-8 chip had very high capture efficiency for bacterial cells. Thus, even when a very small amount of bacterial cells is included in a real sample, the bacterial cells can be efficiently captured.

### Example 9: Effect of buffer pH on cell capture efficiency in three-dimensional structure

A plane structure exhibited the highest cell capture efficiency at a pH of about 4 as shown in Example 1. The three-dimensional microchip 3 manufactured in Example 6 was used to verify again the effect of the pH of 100 mM sodium phosphate. *E. coli* was suspended in sodium phosphate having pH 4 and pH 7, respectively, and the cell capture efficiency was compared. The experiment was repeated three times while flowing buffers at a rate of 300 µℓ/min. The effect of pH was significantly offset compared with the plane structure due to the effect of structure. That is, the method of the present invention can also be used over a broad range of pH.

| | Sodium phosphate pH 4 | Sodium phosphate pH 7 |
|---|---|---|
| 1 | 98.1% | 92.0% |
| 2 | 98.1% | 88.2% |
| 3 | 98.0% | 91.0% |
| Average | 98.0% | 90.4% |

As described above, by simply allowing cells or viruses to be adsorbed to a hydrophobic solid substrate, cells or viruses can be separated. Thus, the cell or virus separation efficiency can be rapidly and simply increased. Further, the cell separation efficiency can be significantly improved using a three-dimensional microstructure and cell separation is possible in a flow control system within several minutes. Moreover, cell separation from a sample such as saliva is possible and the present invention can be applied to the preparation of a sample in a LOC.

## Claims

1. A method of separating cells or viruses using a hydrophobic solid support, comprising contacting a solution containing cells or viruses with a hydrophobic solid support having a water contact angle between 70 and 90 degrees and wherein the hydrophobic solid support has a pillar structure, wherein the contacting is carried out in a fluidic state and the pH of the solution containing cells or viruses is in the range of 2.5 to 4, wherein in the contacting, salt is further added and the concentration of the salt has a range of 0.01 to 2 M, wherein the cells are bacteria.

2. The method of claim 1, wherein the solution containing cells or viruses is saliva, blood, urine, buffer, or a combination thereof.

3. The method of claim 1, wherein an aspect ratio of the pillar has a range of 1:1 to 20:1.

4. The method of claim 1, wherein the ratio of a pillar height (H) to a distance (D) between pillars has a range of 1:1 to 25:1.

5. The method of claim 1, wherein the distance (D) between pillars has a range of 5 to 100 µm.

6. The method of claim 1, wherein the viruses are selected from the group consisting of bacteriophages, plant viruses and animal viruses.

7. The method of claim 1, further comprising washing the hydrophobic solid support having the cells or viruses bound thereto.

8. The method of claim 1, wherein in the contacting, the flow rate of a solution of cells or viruses is 300 µl/min to 900 µl/min.

## Patentansprüche

1. Verfahren zum Trennen von Zellen oder Viren unter Verwendung eines hydrophoben festen Trägers, umfassend das Inkontaktbringen einer Lösung, die Zellen oder Viren enthält, mit einem hydrophoben festen Träger, der einen Wasserkontaktwinkel zwischen 70 und 90 Grad aufweist, und wobei der hydrophobe feste Träger eine Säulenstruktur aufweist, wobei das Inkontaktbringen in einem fluiden Zustand durchgeführt wird und der pH der Lösung, die Zellen oder Viren enthält, im Bereich von 2,5 bis 4 liegt, wobei bei dem Inkontaktbringen außerdem Salz zugegeben wird und die Konzentration des Salzes einen Bereich von 0,01 bis 2 M aufweist, wobei die Zellen Bakterien sind.

2. Verfahren nach Anspruch 1, wobei die Lösung, die Zellen oder Viren enthält, Speichel, Blut, Urin, Puffer oder eine Kombination davon ist.

3. Verfahren nach Anspruch 1, wobei ein Aspektverhältnis der Säule einen Bereich von 1:1 bis 20:1 aufweist.

4. Verfahren nach Anspruch 1, wobei das Verhältnis einer Säulenhöhe (H) zu einem Abstand (D) zwischen Säulen einen Bereich von 1:1 bis 25:1 aufweist.

5. Verfahren nach Anspruch 1, wobei der Abstand (D) zwischen Säulen einen Bereich von 5 bis 100 µm aufweist.

6. Verfahren nach Anspruch 1, wobei die Viren ausgewählt sind aus der Gruppe bestehend aus Bakteriophagen, Pflanzenviren und tierischen Viren.

7. Verfahren nach Anspruch 1, außerdem umfassend das Waschen des hydrophoben festen Trägers, an den die Zellen oder Viren gebunden sind.

8. Verfahren nach Anspruch 1, wobei bei dem Inkontaktbringen die Fließgeschwindigkeit einer Lösung von Zellen oder Viren 300 µl/min bis 900 µl/min beträgt.

## Revendications

1. Procédé de séparation de cellules ou de virus à l'aide d'un support solide hydrophobe, comprenant la mise en contact d'une solution contenant des cellules ou des virus avec un support solide hydrophobe présentant un angle de contact avec l'eau compris entre 70 et 90 degrés et dans lequel le support solide hydrophobe présente une structure en colonne, dans lequel la mise en contact est effectuée dans un état fluide et le pH de la solution contenant des cellules ou des virus est compris dans une plage de 2,5 à 4, dans lequel, pour la mise en contact, du sel est en outre ajouté à une concentration comprise dans une plage de 0,01 à 2 M, dans lequel les cellules sont des bactéries.

2. Procédé selon la revendication 1, dans lequel la solution contenant des cellules ou des virus est de la salive, du sang, de l'urine, un tampon, ou encore une combinaison de ceux-ci.

3. Procédé selon la revendication 1, dans lequel le rapport d'aspect de la colonne est compris dans une plage de 1:1 à 20:1.

4. Procédé selon la revendication 1, dans lequel le rapport de la hauteur de la colonne (H) à une distance (D) entre colonnes est compris dans une plage de 1:1 à 25:1.

5. Procédé selon la revendication 1, dans lequel la distance (D) entre colonnes est comprise dans une plage de 5 à 100 µm.

6. Procédé selon la revendication 1, dans lequel les virus sont sélectionnés parmi le groupe constitué par les bactériophages, les virus de végétaux et les virus d'animaux.

7. Procédé selon la revendication 1, comprenant en outre le lavage du support solide hydrophobe sur lequel sont attachés les cellules ou virus.

8. Procédé selon la revendication 1, dans lequel, lors de la mise en contact, le débit de la solution de cellules ou de virus est compris entre 300 µl/min et 900 µl/min.
